# EUROPÄISCHE PATENTSCHRIFT

(11) **EP 0 468 303 B1**
(45) Veröffentlichungstag und Bekanntmachung des Hinweises auf die Patenterteilung: **28.09.1994**
(21) Anmeldenummer: 91111603.6
(22) Anmeldetag: 12.07.1991
(51) Int. Cl.: C07C 51/48, C07C 53/02

(54) **Verfahren zur Gewinnung von Carbonsäuren aus ihren wässrigen Lösungen**
Method of recovering carboxylic acids from their aqueous solutions
Procédé pour l'obtention d'acides carboxyliques à partir de leurs solutions aqueuses

(30) Priorität: 23.07.1990 DE 4023353
(43) Veröffentlichungstag der Anmeldung: 29.01.1992
(73) Patentinhaber: BASF Aktiengesellschaft, 67063 Ludwigshafen (DE)
(72) Erfinder: Kiefer, Hans, Dr., W-6706 Wachenheim (DE); Reutemann, Werner, Dr., W-6712 Bobenheim-Roxheim (DE)

(56) Entgegenhaltungen:
- EP-A- 0 071 293
- DE-A- 2 545 658
- DE-A- 3 428 319

## Beschreibung

Die vorliegende Erfindung betrifft ein neues und verbessertes Verfahren zur Gewinnung von Carbonsäuren aus ihren wäßrigen Lösungen mit sekundären Amiden, indem die Verluste des sekundären Amids durch Zudosierung des entsprechenden Amins ausgeglichen werden.

Aus der DE-A-25 45 658 ist das Verfahren zur Gewinnung von Carbonsäuren aus ihren wäßrigen Lösungen mit sekundären Amiden bekannt, jedoch mußten bislang die Verluste an sekundärem Amid bei der Extraktion durch Zufügen entsprechender Mengen sekundären Amids ausgeglichen werden.

Der vorliegenden Erfindung lag daher die Aufgabe zugrunde, dem zuvor genannten Nachteil abzuhelfen.

Demgemäß wurde ein neues und verbessertes Verfahren zur Gewinnung von Carbonsäuren der allgemeinen Formel I

R¹-COOH (I),

in der R¹ Wasserstoff, Methyl, Ethyl oder Vinyl bedeutet, durch Extraktion aus ihren verdünnten wäßrigen Lösungen mit einem sekundären Amid der allgemeinen Formel II 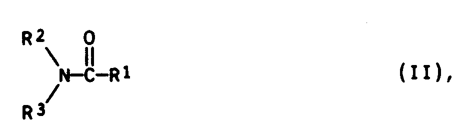
in der R¹ die obengenannten Bedeutungen hat und R², R³ unabhängig voneinander C₁- bis C₈-Alkyl, C₃- bis C₈-Cycloalkyl, C₄- bis C₂₀-Cycloalkylalkyl, Aryl, C₇- bis C₂₀-Aralkyl oder R², R³ gemeinsam eine gegebenenfalls durch C₁- bis C₄-Alkyl ein- bis fünffach substituierte 1,4- oder 1,5-Alkylengruppe bedeuten, gefunden, welches dadurch gekennzeichnet ist, daß man die Verluste an sekundärem Amid II durch Zudosierung des entsprechenden Amins der allgemeinen Formel III 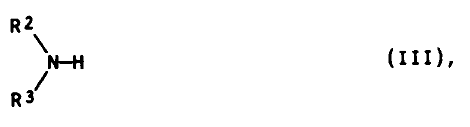
in der R² und R³ die obengenannten Bedeutungen haben, ausgleicht.

Da mit der Carbonsäure I eine Umamidierung stattfinden kann, werden jeweils solche sekundäre Amide II als Extraktionsmittel bevorzugt, in denen der Rest R¹ in den Verbindungen I und II gleich ist. Ameisensäure wird demgemäß zweckmäßigerweise mit einem Formamid, Essigsäure mit einem Acetamid, Propionsäure mit einem Propionsäureamid und Acrylsäure mit einem Acrylsäureamid extrahiert, so daß eine Umamidierung nach außen nicht feststellbar wird. Sollen Gemische verschiedener Säuren, z.B. Ameisensäure und Essigsäure, isoliert werden, so verwendet man bevorzugt die Verbindungen der Formamidreihe als die wirksamsten Vertreter der definitionsgemäßen Klasse.

Hinsichtlich der Amidgruppierungen sind solche Verbindungen II oder auch deren Gemische besonders geeignet, die sich von den sekundären Aminen III wie N-Ethyl-n-cyclohexylamin, N,N-Dicyclohexylamin, N-Methyl-N-benzylamin, N-Methylanilin, N-Ethylanilin, N,N-Diamylamin, N-Methyl-N-2-ethylhexylamin, N-n-Butyl-N-cyclohexylamin, N-Methyl-N-2-heptylamin oder N-Propyl-N-cyclohexylamin ableiten. Am besten haben sich für Ameisensäure die Dibutylformamide, darunter vor allem das Di-n-Butylformamid und für Essigsäure das N-n-Butyl-N-2-ethylhexylacetamid und das N-n-Butyl-N-cyclohexylacetamid bewährt.

Die sekundären Amide II sind entweder bekannt oder nach bekannten Methoden leicht zugänglich und entstehen zusätzlich durch Zugabe von entsprechenden sekundären Aminen III. Liegt ihr Erstarrungspunkt über der Extraktionstemperatur, muß man Gemische der Extraktionsmittel II oder ein Lösungsmittel, vorzugsweise aromatische Kohlenwasserstoffe wie p-Diisopropylbenzol, die mit den Säuren kein Azeotrop bilden, mitverwenden. Bei dieser Arbeitsweise verrringert sich zwar der Wirkungsgrad des Extraktionsmittels II, jedoch bieten sie noch genügend Vorteile im Vergleich zu bekannten Methoden, da die zur Herabsetzung der Erstarrungspunkte normalerweise erforderliche Lösungsmittelmenge mit 10 bis 40 Gew.-%, bezogen auf II, gering ist.

Die Molmenge an zugesetztem sekundärem Amin III beläuft sich auf diejenige Molmenge sekundären Amids, das bei der Extraktion verbraucht wird, kann jedoch auch gesteigert werden, wenn man z.B. das sekundäre Amid II bei der Azeotrop-Destillation auskreist.

Die erforderliche Menge des Extraktionsmittels II hängt von verschiedenen Parametern ab, darunter vor allem von der Temperatur, der Menge und Konzentration der Säure, der Anzahl der Trennstufen sowie den sonstigen apparativen Gegebenheiten, welche die Gleichgewichtseinstellung und damit die Verweilzeit beeinflussen. Hinsichtlich der Art des Extraktionsmittels und der Säure bestehen keine prinzipiellen Unterschiede.

Für die Extraktion kommt vorzugsweise der Temperaturbereich von 0 bis 70°C in Betracht. Bei den unteren Werten dieses Bereiches ist zwar das Aufnahmevermögen des Extraktionsmittels für die Säure größer als bei höheren Temperaturen, dafür ist aber die Geschwindigkeit der Gleichgewichtseinstellung geringer. Das wirtschaftliche Optimum liegt im Temperaturbereich von 20 bis 40°C.

Im Temperaturbereich von 20 bis 40°C benötigt man für die Extraktion von 1 kg Säure 1 bis 10 kg des Extraktionsmittels II und zwar bei Kontaktzeiten von 1 bis 5 Minuten. Bei längeren Kontaktzeiten erniedrigen sich diese Werte, bei kürzeren erhöhen sie sich. Die angegebenen Kontaktzeiten gelten für die Gegenstromextraktion als bevorzugte Ausführungsform, und zwar in einer einfachen Extraktionskolonne ohne sonstige Hilfsmittel wie Blenden, Böden oder Füllkörper, sowie unter der Voraussetzung, daß das Extraktionsmittel mit der geringeren Dichte die zusammenhängende Phase bildet. Durch die Verwendung mehrstufiger Extraktionsapparate, wie z.B. Füllkörper- oder Bodenkolonnen mit vorzugsweise 3 bis 6 theoretischen Böden, erhöht sich der Wirkungsgrad, so daß sich die Menge des Extraktionsmittels nach den bekannten Gesetzmäßigkeiten reduzieren läßt.

Die vorstehenden Angaben beziehen sich auf Säurekonzentrationen von 5 bis 50 Gew.-% der wäßrigen Lösung, wie sie in der Praxis am häufigsten vorkommen. Das Anreicherungsverhältnis ist mit 95 bis 99 Gew.-% ziemlich konstant, d.h. geht man von einer 30 %igen Lösung aus, so verbleiben 0,1 bis 0,3 % Säure im wäßrigen Medium, und unterwirft man eine 10 %ige Lösung der Extraktion, so bleibt eine Lösung mit 0,05 bis 0,1 % Säure zurück. Im allgemeinen ist es am wirtschaftlichsten, die Extraktion so weit zu führen, daß man hierbei eine Mischung aus dem Extraktionsmittel und einer 10 bis 40 gew.-%igen Säure erhält. Aus dieser Mischung destilliert man sodann zunächst das Wasser und danach in einer nachgeschalteten Kolonne die Säure ab. Hierbei kann man es in bekannter Weise auch so einrichten, daß man in der ersten Kolonne nur einen Teil des Wassers entfernt und in der zweiten das restliche Wasser zusammen mit der Säure abdestilliert. In diesem Falle erhält man statt der reinen eine handelsübliche konzentrierte Säure.

Die vorstehenden Darlegungen beziehen sich auf die in der Technik praktisch allein bedeutsame kontinuierliche Herstellung der Carbonsäuren I. Es versteht sich jedoch von selbst, daß das Verfahren gewünschtenfalls auch diskontinuierlich ausgeführt werden kann, wobei die gegebenen Rahmenbedingungen sinngemäß einzuhalten sind.

### Beispiele

### Beispiel 1

In einer Glaskolonne (Durchmesser 28 mm, Höhe 1700 mm, gefüllt mit Glasringen) wurden 798,0 g/h auf 90°C vorgewärmter Zulauf etwa in der Mitte (oberer Kolonnenteil 700 mm, unterer Kolonnenteil 1000 mm) kontinuierlich zugefahren. Der Zulauf setzte sich aus
91,5 g/h Ameisensäure
4,9 g/h Wasser
641,6 g/h Di-n-butylformamid
60,0 g/h Di-n-butylamin
zusammen. Die Destillation wurde im Vakuum (Druck im Destillationskopf 180 mbar) betrieben. Am Kopf der Kolonne wurde mit einem Rücklaufverhältnis von 0,5 ein Gemisch aus Ameisensäure (62,8 g/h) und Wasser (13,1 g/h) abdestilliert (Kopftemperatur ist 59°C). Die Größe des Sumpfvolumens wurde so gewählt, daß die Verweilzeit des Sumpfproduktes bei der Sumpftemperatur von 170°C etwa 1 Stunde beträgt. Aus dem Sumpfvolumen wurde kontinuierlich das Sumpfprodukt mit folgender Zusammensetzung
7,3 g/h Ameisensäure
713,3 g/h Di-n-butylformamid
1,5 g/h Di-n-butylammoniumformiat
abgefahren. Aus der Massenbilanz ergibt sich, daß Di-n-butylamin zu 98 % in Di-n-butylformamid umgesetzt worden ist.

### Beispiel 2

Der Versuchsaufbau entspricht dem Beispiel 1. Der Kopfdruck der Versuchskolonne wurde auf 90 bar gesenkt. Damit ergab sich eine Kopftemperatur von 42°C und eine Sumpftemperatur von 150°C. Die sonstigen Versuchsbedingungen entsprachen Beispiel 1.

Aus der Massenbilanz ergibt sich, daß Di-n-butylamin zu 94 % in Di-n-butylformamid umgesetzt worden ist.

### Beispiel 3

Der Versuchsaufbau entspricht dem Beispiel 1. Der Kopfdruck der Versuchskolonne wurde auf 350 bar erhöht. Damit ergab sich eine Kopftemperatur von 76°C und eine Sumpftemperatur von 190°C. Die sonstigen Versuchsbedingungen entsprachen Beispiel 1.

Aus der Massenbilanz ergibt sich, daß Di-n-butylamin zu >99 % in Di-n-butylformamid umgesetzt worden ist.

### Beispiel 4

Der Versuchsaufbau entspricht dem Beispiel 1. Die Größe des Sumpfvolumens wurde so gewählt, daß die Verweilzeit des Sumpfproduktes bei der Sumpftemperatur von 170°C 30 Min. beträgt. Die sonstigen Versuchsbedingungen entsprachen Beispiel 1.

Aus der Massenbilanz ergibt sich, daß Di-n-butylamin zu 96 % in Di-n-butylformamid umgesetzt worden ist.

### Beispiel 5

Der Versuchsaufbau entspricht dem Beispiel 1. Die Größe des Sumpfvolumens wurde so gewählt, daß die Verweilzeit des Sumpfproduktes bei der Sumpftemperatur von 170°C 2 Stunden beträgt. Die sonstigen Versuchsbedingungen entsprachen Beispiel 1.

Aus der Massenbilanz ergibt sich, daß Di-n-butylamin zu >99 % in Di-n-butylformamid umgesetzt worden ist.

## Patentansprüche

1. Verfahren zur Gewinnung von Carbonsäuren der allgemeinen Formel I
R¹-COOH (I),
in der R¹ Wasserstoff, Methyl, Ethyl oder Vinyl bedeutet, durch Extraktion aus ihren verdünnten wäßrigen Lösungen mit einem sekundären Amid der allgemeinen Formel II 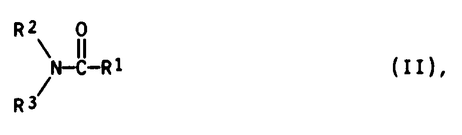 in der R¹ die obengenannten Bedeutungen hat und R², R³ unabhängig voneinander C₁- bis C₈-Alkyl, C₃- bis C₈-Cycloalkyl, C₄- bis C₂₀-Cycloalkylalkyl, Aryl, C₇- bis C₂₀-Aralkyl oder R², R³ gemeinsam eine gegebenenfalls durch C₁- bis C₄-Alkyl ein- bis fünffach substituierte 1,4- oder 1,5-Alkylengruppe bedeuten, dadurch gekennzeichnet, daß man die Verluste an sekundärem Amid II durch Zudosierung des entsprechenden Amins der allgemeinen Formel III 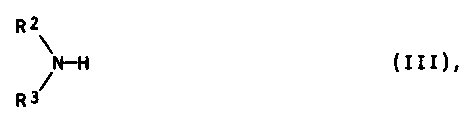 in der R² und R³ die obengenannten Bedeutungen haben, ausgleicht.

## Claims

1. A process for obtaining carboxylic acids of the general formula I
R¹-COOH (I)
where R¹ is hydrogen, methyl, ethyl or vinyl, by extraction from their dilute aqueous solutions with a secondary amide of the general formula II 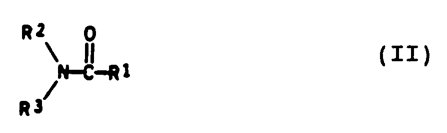 where R¹ has the abovementioned meanings and R² and R³ independently of one another are each C₁-C₈-alkyl, C₃-C₈-cycloalkyl, C₄-C₂₀-cycloalkylalkyl, aryl or C₇-C₂₀-aralkyl, or R² and R³ together form a 1,4- or 1,5-alkylene group which is unsubstituted or monosubstituted to penta-substituted by C₁-C₄-alkyl, wherein the losses of secondary amide II are compensated by metering in the corresponding amine of the general formula III 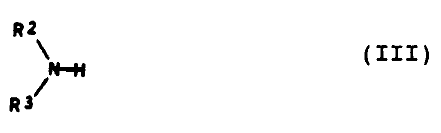 where R² and R³ have the abovementioned meanings.

## Revendications

1. Procédé d'obtention d'acides carboxyliques de la formule générale I
R¹-COOH (I),
dans laquelle R¹ représente un atome d'hydrogène, le radical méthyle, éthyle ou vinyle, par extraction à partir de leurs solutions aqueuses diluées, avec un amide secondaire de la formule générale II 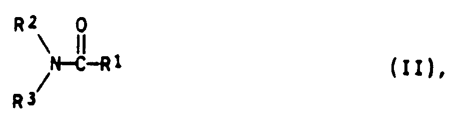 dans laquelle R¹ possède les significations qui lui ont été attribuées ci-dessus et R² et R³ représentent, chacun indépendamment l'un de l'autre, un radical alkyle en C₁ à C₈, cycloalkyle en C₃ à C₈, cycloalkyl(C₄-C₂₀)alkyle, aryle, alkylaryle(C₇-C₂₀), ou bien R² et R³ forment ensemble un groupe 1,4- ou 1,5-alkylène éventuellement substitué de 1 à 5 fois par des radicaux alkyle en C₁ à C₄, caractérisé en ce que l'on compense les pertes en amide secondaire II par l'introduction dosée de l'amine correspondante de la formule générale III 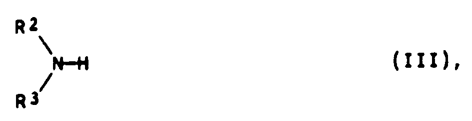 dans laquelle R² et R³ possède les significations qui leur ont été attribuées ci-dessus.
